Europäisches Patentamt

European Patent Office

Office européen des brevets

(11) Publication number: **0 176 690**
**A1**

(12) **EUROPEAN PATENT APPLICATION**

(21) Application number: 85109467.2

(22) Date of filing: 27.07.85

(51) Int. Cl.⁴: **C 07 D 311/72**
**C 07 B 37/04**
**//A61K31/355**

(30) Priority: 18.09.84 US 651726

(43) Date of publication of application:
09.04.86 Bulletin 86/15

(84) Designated Contracting States:
AT BE CH DE FR GB IT LI LU NL SE

(71) Applicant: HENKEL CORPORATION
7900 West 79th Street
Minneapolis Minnesota 55435(US)

(72) Inventor: Swanson, Ronald R.
Route 1
Taylors Falls Minnesota 55084(US)

(74) Representative: von Kreisler, Alek, Dipl.-Chem. et al,
Deichmannhaus am Hauptbahnhof
D-5000 Köln 1(DE)

(54) Vapor/liquid phase methylation of non-alpha-tocopherol.

(57) This invention relates to the production of alpha-tocopherol from non-alpha-tocopherol homologues by contacting the non-alpha-tocopherol homologue with an alkylation agent such as methanol, formaldehyde, or dimethyl ether for a sufficient length of time at a temperature in the range of from about 300° Centigrade to about 550°C in the presence of an alkylation catalyst. Methylation will occur in a substantial amount, producing alpha-tocopherol.

EP 0 176 690 A1

# VAPOR/LIQUID PHASE METHYLATION OF NON-ALPHA-TOCOPHEROL

## BACKGROUND OF THE INVENTION

This invention relates to the methylation of beta-, gamma- and delta-tocopherols thereby increasing their vitamin E activity. Alpha tocopherol, having the greatest vitamin E activity, can be produced through the process of this invention, by the methylation of these non-alpha-tocopherols.

The term "vitamin E" was originally used to designate the active component of certain vegetable oils. Vitamin E activity means the physiological activity of a group of nutrient materials originally isolated from various natural sources. The materials having vitamin E activity all belong to a distinct series of compounds which are all derivatives of chroman-6-ol. These compounds are all tocol derivatives having an isoprenoid $C_{16}$-side chain. The term "tocol" is used to mean 2-methyl-2-(4',8',12'-trimethyltridecyl)chroman-6-ol. The nomenclature currently used for 5,7,8-trimethyl tocol is alpha-tocopherol; 5,8-dimethyl tocol is beta-tocopherol; 7,8-dimethyl tocol is gamma-tocopherol; and 8-methyl tocol is delta-tocopherol. The tocopherol nomenclature will be used throughout.

The compounds of the most importance for vitamin E activity are the more highly methylated tocols, especially 5,7,8-trimethyl tocol. The physiological activity of this group of compounds is measured by the ability to maintain fertility in rats. The vitamin E active compounds have a variety of beneficial effects in humans with the alpha-tocopherol homologue having the highest potency as measured by its effectiveness in maintaining rat fertility. For this reason, the term vitamin E is frequently used inter-changeably with d-alpha-tocopherol, the naturally occurring tocopherol of highest vitamin E activity.

The tocopherols are found widely distributed in normal foods, occurring in the highest concentration in the cereal grain oils, principally in corn and wheat oils, but also in barley and rye. They are also found in vegetable oils such as safflower, soybean, peanut, cottonseed, linseed, sunflower, rapeseed and palm, and in other vegetable sources, e.g. palm leaves, lettuce, alfalfa, rubber latex and a variety of other plant materials. The concentration of the most active form, the d-alpha-tocopherol varies widely among the different sources. Two of the highest sources of d-alpha-tocopherol are safflower oil and sunflower oils, although a commonly available source is soybean oil, which has a considerably lower percent of d-alpha-tocopherol with significantly higher percentages of the gamma- and delta-homologues.

The naturally occurring tocopherols are generally isolated from natural products such as vegetable oil sources by various combinations of such procedures as esterification, saponification, hydrogenation, extraction, distillation, ion-exchange, absorption chromatography, precipitation of sterols, crystallization, and many others. The tocopherol concentrate isolated in this manner will vary in composition depending on the particular vegetable source and separation techniques used. Generally, however, the concentrate is a mixture of tocopherol homologues containing about 40% or more impurities such as residual sterols, hydrocarbons, and fatty acids. The non-alpha-tocopherol in the mixture can be converted to the more biologically active alpha-tocopherol by introducing methyl substituents into the aromatic ring (Tocol ring). A variety of ways are known for achieving this upgrading. Presently known methods for upgrading beta-, gamma- and delta-tocopherols by methylation include halomethylation, aminomethylation, hydroxymethylation or formylation to introduce a methyl functional group followed by reduction to give the methylated tocopherol.

Aminomethylation is described, for example, in Weisler U.S. Patent Nos. 2,486,539 and 2,519,863. These methods are generally applied to low potency tocopherols extracted from plant sources. The mixed tocopherols are reacted with an amino-alkylating agent, suitably piperidine hydrochloride and paraformaldehyde in an ethanol solvent acidified with hydrochloric acid. The amino alkyl addition product of mixed tocopherols is then reduced by catalytic hydrogentation to convert the non-alpha-tocopherols in the mixture to alpha tocopherol.

Another methylation method is halomethylation. The halomethylation reaction is preferably a chloromethylation achieved by reaction of the non-alpha-tocopherols present with a solution of formaldehyde in the presence of hydrogen chloride. This chloromethyl group is converted into a methyl group by a reduction procedure.

Formylation methods are described in U.S. Patent No. 2,592,531 in which mixed tocopherols are reacted with formaldehyde in the presence of an organic acid catalyst. Other acid catalysts such as phosphoric and boric acid can also be utilized. More recently, Japanese Patent No. 79-143,054 describes a process for preparing alpha-tocopherol from mixed tocopherols by reacting with formaldehyde and a reducing agent in the presence of an organic acid, to convert the non-alpha-tocopherols directly to alpha-tocopherols by formylating and reducing in a single stage.

Other more direct methods of methylation are applied to aromatic compounds which result in the addition of a methyl group to the aromatic ring. One such method is described in U. S. Patent No. 2,448,942 which discloses a process for methylating phenols using alcohol or methyl ether in the vapor phase over various metal oxide catalysts, such as aluminum oxide, thorium oxide, zirconium oxide, zinc oxide, iron oxide, chromium oxide, barrium oxide, manganese oxide, magnesium oxide and calcium oxide. This process uses super atmospheric pressures and temperatures in the range of 300 to 450° Centigrade.

Another similar process is described in U.S. Patent 3,718,704 which provides for the methylation of substituted and unsubstituted mono- and polynuclear ring aromatic compounds. This process uses a carbon-oxide containing reactant gas in the presence of a catalyst selected from either a group IB, group IIB, or group VIB metal oxide. In accordance with that process, the reaction is carried out at temperatures between 400 and 1,200° F with pressures of from 0 to 15,000 psig.

Heretofore, methods for methylation of tocopherol have maintained the temperatures lower than the temperatures necessary for the non-acidic vapor/liquid methylation of the instant invention. U.S. Patent 4,239,691, for example, describes a process for the hydroxymethylation-hydrogenation of non-alpha-tocopherols. This patent states that the reaction temperature should not be so high as to cause decomposition of the reactants of products and is preferably held below 300°C.

It is an object of the instant invention to describe a process for the direct, one step vapor/liquid phase methylation of tocopherols with a methylating agent in the presence of metal oxide catalysts, but in the absence of an acid. It is a further object of the instant invention to achieve the vapor/liquid phase methylation of non-alpha-tocopherols without substantial degradation of the tocopherol compounds. Still another object is to provide a method for the direct methylation of non-alpha-tocopherols without the necessity of a reduction or hydrogenation step. A further object is to increase vitamin E activity of less methylated tocopherol homologues by the addition of one or more methyl groups to these homologues. Other objects will become apparent as this description proceeds.

## BRIEF DESCRIPTION OF THE INVENTION

The vitamin E activity of non-alpha-tocopherols can be improved by direct methylation, producing methylated tocopherols, a substantial portion of which is alpha-tocopherol. This methylation can be achieved by contacting the non-alpha-tocopherol homologues with a methylating agent in the presence of an alkylation catalyst at a temperature in the range of from about 300 to about 550°C (Centigrade) for a sufficient length of time. In accordance with this process, a feed material containing non-alpha-tocopherols is subjected to a direct methylation in a vapor/liquid phase condition whereby non-alpha-tocopherols are directly methylated to produce a methylated tocopherol product, a substantial portion of which is alpha-tocopherols. The alpha-tocopherols are produced by the addition of a single methyl group to either beta- or gamma-tocopherol; or by the addition of two methyl groups to the delta-tocopherol. Delta-tocopherol can be upgraded to beta-gamma-tocopherol by the addition of a single methyl group.

Surprisingly, it has been found that, even at the temperatures necessary for the instant invention, from about 300 to about 550° Centigrade, only a small portion of the tocopherols will be degraded. Moreover, less methylated tocopherols can be methylated thereby increasing the vitamin E activity of the methylated product, a substantial portion of which is alpha-tocopherol.

A methylating agent is a material capable of providing or releasing a methyl group to the unsubstituted aromatic positions of the non-alpha-tocopherols. Notably these positions are the 5 and 7 positions of the delta-tocopherol, the 7 position of the beta-tocopherol, and the 5 position of the gamma-tocopherol aromatic ring. Preferred methylating agents which are capable of directly providing a methyl group under the conditions of this reaction are methanol, dimethyl ether, formaldehyde and dimethyl carbonate.

The tocopherol and the methylating agent are contacted in the presence of an alkylation catalyst. Such a catalyst is any catalyst which is capable of inducing an alkylation reaction. Typically these catalysts are metal oxides. Such catalysts can be selected from the metal oxides and mixtures thereof of the metals selected from groups IIA, IIIA, IVA, IVB, VB, VIB, VIIB, VIII and IIB of the Periodic Table of Elements.

Non-alpha-tocopherols have at least one hydrogen, present in a substitutable position on their aromatic ring, and, if they are subjected to temperatures in the stated range in the presence of a methylating agent and an alkylation catalyst, methylation will occur within a short period of time. Moreover, the length of time necessary for methylation to occur at these temperatures is sufficiently short to permit a significant amount of a non-alpha-tocopherol feed material to be methylated before a significant amount of degradation occurs.

After methylation the more highly methylated tocopherol product can be collected, and used, or subjected to further methylations in order to increase the alpha-tocopherol content. If impurities are present, they may also be separated at this point. Preferably, after methylation alpha-tocopherol is separated and not subjected to further methylations and any remaining non-alpha-tocopherols can be recycled for additional methylation.

## DETAILED DESCRIPTION OF THE INVENTION

Beta-, gamma- and delta-tocopherol homologues can be methylated to increase their vitamin E activity. Natural organic sources such as vegetable oils and plant materials can be used to provide the organic feed materials containing the beta-, gamma- and delta-tocopherols for the instant invention. There is no critical minimum non-alpha-tocopherol concentration level necessary for the instant invention. Even materials as low as 5% by weight in non-alpha-tocopherol homologues can be used as starting materials for this process. Natural organic sources, such as vegetable oils and plant materials can be used as the beta-, gamma- and delta-tocopherol containing feed material for this invention. Representative but nonexhaustive examples of suitable substances are: safflower, soybean, peanut, cottonseed, linseed, sunflower, rapeseed and palm oils. The starting material can also be taken from other plant sources such as palm leaves, lettuce, alfalfa, rubber latex, and a variety of other plant materials.

It would, however, be more practical to employ a feed material having substantial non-alpha-tocopherol concentrations. Acceptable feed material for the instant invention would have a minimum non-alpha-tocopherol concentration of about 25% by weight beta-, gamma- and delta-tocopherols. A more preferred feed material would have a minimum of about 40% by weight beta-, gamma- and delta-tocopherols. Frequently, vegetable oils are used to produce a concentrate that is up to 60% mixed tocopherols.

A more preferred feed material would be a highly purified tocopherol containing only beta-, gamma-, and delta-tocopherol so as to simplify the separation of alpha-tocopherol from the product issuing from the reactor so as to allow recycle of the unconverted beta-, gamma-, and delta-tocopherols.

The instant invention can be used to improve the vitamin E activity by methylating the non-alpha-tocopherols in such materials. The instant invention can be used in a batch or continuous manner, and should be conducted in an inert oxygen-free atmosphere. If desired, a carrier material can be used with the non-alpha-tocopherol-containing feed material and/or with the methylating agent or the two together if they are combined. Preferably these are all added simultaneously. Representative examples of such suitable inert carriers are: nitrogen, argon, helium, hydrogen, and neon. Even non-reactive hydrocarbons, such as methane, ethane, propane, butane and pentane can be used.

Preferably the instant invention is carried out under ambient pressures, although it is permissible to use super- or sub-atmospheric pressures. Pressures up to 225 atmospheres or in the range of from about 0.1 ATM to about 225 ATM can be used.

The minimum temperature necessary for the instant invention is about 300° C. The maximum temperature permitted is 550°C. The temperature should not be permitted over this range in view of the larger and more rapid degradation rate of the tocopherols. Preferably the instant process is conducted at temperatures in the range of from about 350°C to about 500°C. Most preferably, the temperatures are maintained in the range of from about 390°C to about 470°C. The beta-, gamma- and delta-tocopherols, together with the methylating agent and the alkylation catalyst, should be subjected to the required temperature for a sufficient length of time to cause methylation of the non-alpha-tocopherols. The danger of substantial tocopherol degradation is minimal. Even at 470°C, for example, the amount of tocopherol degraded per second is less than 2% by weight. Non-alpha-tocopherol feed material could be methylated at temperatures of from about 390 to about 470°C for a period of time up to 8 seconds without substantial degradation of the tocopherol in the feed. Even at 470°C, methylation of beta-, gamma- and delta-tocopherols can be achieved in a period of time well under one second, but a longer reaction time will permit a greater degree of methylation to occur.

The use of the instant process to produce tocopherols having an increased vitamin E activity, a substantial portion of which is alpha-tocopherol, therefore, can allow for the methylation of the tocopherol homologues at temperatures between 300 and 550°C for a sufficient length of time. A reaction time of less than 5 sec. is preferred. In order to minimize tocopherol degradation, however, a preferred application of the instant invention would minimize the length of time that the tocopherol homologues are reacted at these temperatures, especially temperatures from 440°C to 550°C.

Repeat methylation can be done to further methylate non-alpha-tocopherols remaining after one vapor/liquid phase methylation. Thus, the methylated tocopherol product of one reaction can suitably be subjected to one or more additional methylations in accordance with the instant invention. Most preferably, after one or two methylations, remaining non-alpha-tocopherol homologues are separated by any convenient method from the alpha-tocopherol and the non-alpha-tocopherol homologues are then recycled for another vapor/liquid phase methylation. Depending upon the feed material used, certain amounts of impurities such as sterols could be present in the methylated product. These impurities can likewise be separated from the tocopherol product. Such separations can be accomplished by methods such as distillation, crystallization, adsorption, and extraction. Preferably the beta-, gamma- and delta-tocopherol homologues are contacted with a methylating agent in the presence of an alkylation catalyst for a length of time less than one second and most preferably for less than 0.5 seconds.

It may be acceptable, in certain applications, however, to permit the beta-, gamma- and delta-tocopherol homologues to be exposed to such temperatues for longer periods of time in spite of slightly increased degradation losses. For example, limiting contact time to less than 5 seconds keeps degradation at an acceptable minimum of less than 10% by weight of the tocopherol even at 550°C.

The methylating agent is the source of the methyl group which is added to the non-alpha-tocopherols. Preferred methylating agents used in the instant invention to achieve direct methylation of beta-, gamma- and delta-tocopherols are formaldehyde, methanol, dimethyl carbonate, and dimethyl ether. The most preferred of these is methanol. Acceptably the methylation agent is present in a minimum amount of at least one equivalent per equivalent of non-alpha-tocopherol. An acceptable concentration range is from about 1 to about 25 moles of the methylating agent per equivalent of the non-alpha-tocopherols. Most preferably, the methylating agent is present in an amount of from about 1.35 to about 15 moles per equivalent of the non-alpha-tocopherols present.

The methylating agent and the non-alpha-tocopherol may be added separately to the reactor, but simultaneously, or they may be premixed and added as a solution. Separate addition allows the ratio of methylating agent to non-alpha-tocopherol to be changed conveniently at any time during the run, this being most advantageous at the beginning and end of a run. Most preferably, the tocopherol is diluted with at least a portion of the methylating agent (if it is a liquid) to reduce the viscosity and to give a better dispersion of the non-alpha-tocopherol in the reactor with the remaining methylating agent being added separately. The use of methanol along with the tocopherol and dimethyl ether added separately is an example of this. More preferably, the methylating agent and the feed material are pre-mixed before addition to the reaction vessel. Whatever manner of addition is used,an inert carrier material (inert gas) can be added.

A great many catalysts are capable of inducing the alkylation reaction. Any one or more of these catalysts would be suitable for use with the instant invention. Alkylation catalysts can be selected from the metal oxides of the metals selected from groups IIA, IIIA, IVA, IVB, VB, VIB, VIIB, VIII and IIB of the Periodic Table of Elements. Of these metal oxides, a preferred catalyst can be any metal oxide or oxides of a metal or metals selected from the group consisting of: Be, Mg, Ca, Ti, Zr, V, Mo, Cr, Mn, Tc, Fe, Co, Ni, Zn, Cd, In, Sn, Si, Al, La, Ce, Pr, and Nd.

The alkylation catalysts called for by the instant process can be neat or supported on an inert material and can be prepared by any convenient method. Such preparation can furthermore be external to the methylation vessel or can be an in situ preparation.

One such example of an in situ catalyst preparation is to load the vapor/liquid phase methylation vessel with a metal salt of the metals selected from the groups IIA, IIIA, IVA, IIB, IVB, VB, VIB, VIIB, and VIII of the Periodic Table of Elements. The metal salt is then reacted or decomposed to the metal oxide. This metal oxide is then left in the methylation vessel, the by-products and any remaining reactants are removed, and the vessel is then ready for methylation.

The instant invention will be more fully understood from the examples which follow. These examples are intended to clarify the instant invention and not to limit it. All parts and percentages are by weight unless otherwise specified.

## EXAMPLES I (PART A)

Catalyst Preparation - a $V_2O_5$, $SiO_2$, and SnO catalyst.

Materials:

NH$_4$VO$_3$-70.3 grams (0.6 moles)

Oxalic acid (CO$_2$H)$_2$-48.8 grams (gm.)

Tetraethylorthosilicate (Et)$_4$ SiO$_4$-6.3 gm.

SnSO$_4$-12.9 gm.

Procedure:

Si(OH)$_4$ was made by hydrolizing 6.3 gm. of a tetraethylorthosilicate in 300 mls. of 0.1 NHNO$_3$. This mixture was stirred magnetically at room temperature for 18 hours.

Sn(OH)$_2$ was made by added 12.9 gm. SnSO$_4$ to 258 gm. of NH$_4$NO$_3$ (1:20 volume ratio). This was stirred on a magnetic stirrer and heated for one half hour at about 75°C to evenly disperse the tin sulfate. The pH was measured at 0.7. This was neutralized with about 40 mls. of 28% NH$_4$OH and the precipitate was washed 4 times with 300 mls. of water. The precipitate was placed in an oven overnight to dry at 80°C. 8.3 gm. of Sn(OH)$_2$ resulted.

#3 - 70.3 gm. of NH$_4$VO$_3$ and 48.8 gm. of (CO$_2$H)$_2$- oxalic acid - was mixed in 300 mls. of water and stirred magnetically for one half hour. Si(OH)$_4$ solution added and Sn(OH)$_2$ with stirring. The precipitate was dried overnight in 110°C oven and was then crushed and sieved to 10-24 mesh particles. There was a yield of 57.5 gm. of the catalyst.

Calcination was at 400°C for three hours in an airstream, giving the $V_2O_5$, $SiO_2$ and SnO catalyst.

The powder obtained from calcination was then pressed into short cylinders or discs, $5/16$" x $3/16$"

thick.

## EXAMPLE I (PART B)

Description of Reactor Used For Vapor/Liquid Phase
Methylations.  A one inch internal diameter stainless
steel verticle tube reactor was used.  This tube was
packed with 5" of 3 mm. glass beads on the top with 1"
of the catalyst in a layer below the 5" of glass beads,
and then underneath the catalyst layer was another 3"
of 3 mm. glass beads supported by a wire screen.  Three
heating elements were used and were clamped vertically
in succession around the outside of the tube.  A feed
input line was attached to the top of the tube.  The
tube was closed to the outside atmosphere.  A nitrogen
inlet line was attached to the feed inlet line so that
a nitrogen gas carrier could be used during the reaction.  A thermal well permitting temperature readings
ran from the top of the reactor down to the wire
screen.  Immediately below the wire screen, cooling
coils surrounded the bottom outlet of the reactor.  A
collection flask was attached below these cooling coils
to collect the product.

## EXAMPLE I (PART C)

The Vapor/Liquid Phase Methylation.  The catalyst
prepared in part A of this Example was $V_2O_5$, SnO, $SiO_2$
in a 20:2:1 molar ratio.  This catalyst was placed in
the 1" diameter stainless steel fixed bed reactor in a
layer 1" deep.

Methanol, the alkylation agent, and a tocopherol
concentrate were then pumped into the reactor in a 1:10
mole ratio of alpha-, beta-, delta-, and gamma-tocopherol to methanol.  The tocopherol concentrate had the
following composition:  18.35% by weight delta tocopherol;  38.9% by weight beta, gamma tocopherol; and
5.25% by weight alpha tocopherol.  A nitrogen gas carrier was used.  During the tocopherol methylation, the following data was recorded:

### First period:

| Time in min. | Temp.(°C) | Feed Rate | Event |
|---|---|---|---|
| 0 | 380 | 1:1.50 gm./min. | Start to pump methanol and tocopherol |
| 5 | 450 | 0.78 gm./min. | Measure flow |
| 50 | 370 | 0.78 gm./min. | Product began leaving the reactor |
| 65 | 400 | 1.72 gm./min. | Methylated tocopherol Sample No. 1 |
| 80 | 405 | 1.72 gm./min | Tocopherol produce Sample No. 2 |

### Second period:

| Time in min. | Temp.(°C) | Feed Rate | Event |
|---|---|---|---|
| 0 | 375 | 1.38 gm./min. | Changed tocopherol and methanol at 1:10 mole ratio |
| 32 | 400 | 1.38 gm./min. | Product exiting reactor |
| 42 | 400 | 2:02 gm./min. | Measure flow rate |
| 47 | 405 | | Lower flow rate |
| 57 | 395 | 1.74 gm./min. | Measure flow rate |
| 67 | 415 | | Lower flow rate |

| Time in min. | Temp.(°C) | Feed Rate | Event |
|---|---|---|---|
| | | | Measure flow rate, Took tocopherol product |
| 82 | 400 | 1.46 gm./min. | Sample No. 3 |
| 112 | 410 | 1.46 gm./min. | Sample No. 4 |
| 142 | 380 | 1.46 gm./min. | Sample No. 5 |
| 172 | 400 | 1.46 gm./min. | Sample No. 6 |

All tocopherol samples taken were submitted for gas chromatograph analysis and the following data resulted:

| Number | % (by wt.) Delta-tocopherol | % (by wt.) Beta-gamma-tocopherol | % Alpha-tocopherol |
|---|---|---|---|
| 1 | 11.1 | 28.7 | 15.0 |
| 2 | 12.4 | 30.2 | 13.9 |
| 3 | 13.4 | 32.9 | 11.7 |
| 4 | 13.2 | 32.9 | 11.9 |
| 5 | 12.5 | 32.4 | 12.8 |
| 6 | 13.0 | 33.1 | 12.5 |

These results indicated an increase in alpha tocopherols, and a simultaneous decrease in both delta- and beta-gamma tocopherols indicating that alkylation took place.

## EXAMPLE II (PART A)

Preparation of a $V_2O_5$ and $TiO_2$ alkylation catalyst
Materials:

$NH_4VO_3$ - 70.3 gm. (0.6 moles)
Oxalic acid - 48.8 gm.
$TiO_2$ - 47.9 gm. (0.6 moles)

Procedure:

NH$_4$VO$_3$ and oxalic acid were mixed in 300 mls. of water with stirring for about one-half hour. TiO$_2$ was added while continuing magnetic stirring. Metal compounds and oxalic acid were well mixed and they were put into a 110°C oven for 24 hours. After this, a slurry was made with a small amount of water, and the contents were well mixed. The mixture was again placed in an oven and dried.

The dried product was ground and sieved to mesh size 10-24, and this was then calcined at 400°C for three hours in an airstream to activate the catalyst.

The powder obtained from calcination was then pressed into short cylinders or discs, $^5/_{16}$" x $^3/_{16}$" thick.

## EXAMPLE II (PART B)

The catalyst prepared under part A was used in the same fixed bed reactor described in sample I.

Methanol and a tocopherol concentrate were pumped into the reactor at a ratio of 10 moles of methanol per mole of non-alpha-tocoherol. The tocopherol concentrate used was analyzed to contain 5.3% by weight alpha-tocopherol, 41.35% by weight beta-gamma-tocopherol, and 18.8% delta-tocopherol. The following data was recorded during tocopherol methylation:

| Time in min. | Temp. (°C) | Feed rate | Event |
|---|---|---|---|
| 0 | 400 | 0.67 g./min. | Began to charge methanol and tocopherol feed |
| 25 | 400 | 1.58 g./min. | |
| 45 | 405 | 1.28 g./min. | Sample No. 1 |
| 75 | 395 | 1.12 g./min. | Sample No. 2 |
| 105 | 400 | 1.45 g./min. | Sample No. 3 |
| 135 | 390 | 1.45 g./min. | Sample No. 4 |
| 145 | | | Cooled down reactor |

The samples were submitted for analysis by gas chromatograph and the following product composition was found:

| Sample Number | % (by wt) Delta-tocopherol | % (by wt.) Beta-gamma-tocopherol | % Alpha-tocopherol | % Sterols |
|---|---|---|---|---|
| 1 | 14.1 | 34.1 | 11.1 | 3.3 |
| 2 | 15.3 | 35.5 | 9.3 | 3.7 |
| 3 | 15.2 | 35.6 | 9.0 | 3.6 |
| 4 | 15.3 | 35.9 | 9.1 | 3.7 |

The product analyzed indicated an increase in alpha-tocopherols and a simultaneous decrase in both delta-tocopherols and beta-gamma-tocopherols indicating that alkylation took place.

## WHAT IS CLAIMED

1. The methylation of a non-alpha-tocopherol homologue by a process comprising: contacting the non-alpha-tocopherol homologue with a methylating agent in the presence of an alkylation catalyst at a temperature in the range of from about 300 to about 550°C for a sufficient length of time thereby producing methylated tocopherol.

2. A process as described in claim 1 wherein the alkylation catalyst is a metal oxide of a metal selected from the group consisting of the following groups of the Periodic Table of Elements: IIA, IIIA, IVA, IIB, IVB, VB, VIIB, and VIII.

3. A process as described in claim 2 wherein the alkylation catalyst is a metal oxide of a metal selected from the group consisting of: Be, Mg, Ca, Ti, Zr, V, Mo, Cr, Mn, Tc, Fe, Co, Ni, Zn, Cd, In, Sn, Si, Al, La, Ce, Pr and Nd.

4. A process as described in claim 1 wherein the methylating agent is selected from the group consisting of methanol, formaldehyde, dimethyl ether, and dimethyl carbonate.

5. A process as described in claim 4 wherein the methylating agent is present in a minimum concentration of from about one to about 25 equivalents per equivalent of methylatable sites on the non-alpha-tocopherol.

6. A process as described in claim 1 wherein the temperature is in the range of from about 390°C to about 470°C.

**0176690**

7. A process as described in claim 1 wherein the length of time is a maximum of 5 seconds preferably.

8. A process as described in claim 1 wherein a carrier material is used from the group consisting of nitrogen, argon, helium, and neon.

9. A process as described in claim 1 wherein alpha-tocopherol is separated from the methylated tocopherol produced and the remaining non-alpha-tocopherol homologues are subjected to another methylation by contacting the non-alpha-tocopherol homologue with a methylating agent in the presence of an alkylation catalyst at a temperature in the range of from about 300°C to about 550°C for a sufficient length of time.

10. A process as described in claim 1 wherein the alkylation catalyst is supported on an inert material.

| Category | Citation of document with indication, where appropriate, of relevant passages | Relevant to claim | CLASSIFICATION OF THE APPLICATION (Int. Cl.4) |
|---|---|---|---|
| | **DOCUMENTS CONSIDERED TO BE RELEVANT** | | EP 85109467.2 |
| D,A | US - A - 4 239 691 (NELAN et al.)<br>* Claims 1,7,8 *<br>-- | 1,4,6 | C 07 D 311/72<br>C 07 B 37/04<br>//A 61 K 31/35 |
| D,A | US - A - 2 519 863 (L. WEISLER)<br>* Column 2, lines 17-22; claims 1-3; column 2, lines 34-54 *<br>-- | 1,4,6 | |
| A | US - A - 3 338 922 (K. SHIZUMASA et al.)<br>* Claim 1 *<br>---- | 1,4,6 | |
| | | | **TECHNICAL FIELDS SEARCHED (Int. Cl.4)**<br><br>C 07 D 311/00 |

The present search report has been drawn up for all claims

| Place of search | Date of completion of the search | Examiner |
|---|---|---|
| VIENNA | 21-01-1986 | BRUS |